# EUROPEAN PATENT APPLICATION

(11) **EP 4 799 637 A1**
(43) Date of publication of application: **02.09.2026**
(21) Application number: 24896261.5
(22) Date of filing: 12.11.2024
(51) Int. Cl.: A61K 38/26, A61K 9/08, A61P 3/10, A61P 3/04

(54) **NASAL ADMINISTRATION COMPOSITION CONTAINING GLP-1 RECEPTOR AGONIST, AND PREPARATION METHOD THEREFOR AND USE THEREOF**

(30) Priority: 01.12.2023 WO PCT/CN2023/135780
(71) Applicant: Zhongshan Wanhan Pharmaceutical Co., Ltd., Zhongshan, Guangdong 528451 (CN)
(72) Inventor: WANG, Heran, Zhongshan, Guangdong 528451 (CN); HUANG, Guanbin, Zhongshan, Guangdong 528451 (CN); DU, Zhibo, Zhongshan, Guangdong 528451 (CN); WANG, Likun, Zhongshan, Guangdong 528451 (CN); WANG, Tao, Zhongshan, Guangdong 528451 (CN); HUANG, Weixiong, Zhongshan, Guangdong 528451 (CN); LI, Tao, Zhongshan, Guangdong 528451 (CN); WANG, Guanhua, Zhongshan, Guangdong 528451 (CN); PENG, Wei, Zhongshan, Guangdong 528451 (CN)
(74) Representative: Bayramoglu et al.
(86) International application number: PCT/CN2024/131548
(87) International publication number: WO 2025/113170

(57) **Abstract**

The present invention belongs to the field of medical technologies, in particular to a composition for nasal administration comprises a GLP-1 receptor agonist, and a preparation method and use thereof. Both in vivo and in vitro tests show that the composition of the present invention has good stability, rats have good bioavailability after nasal administration, and the drug distribution in brain tissues is very small.

## Description

### TECHNICAL FIELD

The present invention belongs to the field of medical technologies, in particular to nasal administration composition containing glp-1 receptor agonist, and preparation method therefor and use thereof.

### BACKGROUND TECHNOLOGY

Diabetes mellitus (DM) is one of the most common chronic diseases. China is the country with the largest number of diabetic patients in the world, and the prevalence rate of diabetic patients in adults has been as high as 11.9%, of which type 2 diabetes mellitus (T2DM) accounts for about 90%, and the prevalence rate of obese and overweight people with diabetes mellitus has increased significantly. T2DM is caused by a combination of β islet cell dysfunction, defective insulin action, and abnormal glucagon secretion. Glucagon-like peptide-1 (GLP-1) receptor agonists are a new class of hypoglycemic drugs in the field of T2DM treatment, which can significantly improve some key pathophysiological defects of T2DM, and have additional benefits except for a hypoglycemic effect such as reducing cardiovascular death, improving atherosclerosis, reducing body weight, reducing systolic pressure, and improving blood lipid profiles, bringing new hope to patients with T2DM. The development of GLP-1 receptor agonists has gone through a development process from short-acting (2-3 injections per day) to long-acting (1 injection per day) to ultra-long-acting (1 injection per week). At present, GLP-1 receptor agonists on the market worldwide are all systemically administered, most by injection, among which semaglutide is administered orally.

Although an administration route of GLP-1 receptor agonists has been greatly improved, the inherent defects of systemic administration still cause great inconvenience to patients with diabetes mellitus or overweight/obesity who require long-term medication, so there is still a need to develop an administration route that is more consistent with the patient's compliance.

Nasal administration has the advantages of rapid absorption, rapid onset of action, avoidance of hepatic first-pass effect, high bioavailability, ease of use, and direct delivery of drugs into the brain by bypassing the blood-brain barrier. Although a variety of polypeptide products administered through nasal administration have been successfully marketed or entered clinical trials, the nasal absorption of drugs is a very complex process, for example, Huang Zhuang, et al. pointed out in the article "Research Progress on Nasal Administration of Polypeptide Drugs" that the factors affecting the nasal absorption of drugs include but are not limited to: drug properties (e.g., relative molecular weight, and lipophilicity), preparation characteristics (e.g., pH, osmotic pressure and viscosity of a preparation), intranasal environment (e.g., enzyme activity, cilia clearance, and nasal mucus), so there is great uncertainty about whether a particular nasal absorption enhancer can achieve intranasal absorption of a certain polypeptide.

CN116942612A discloses a method for nasal spray absorption of GLP-1 receptor agonists such as semaglutide using caprylocaproyl macrogolglyceride, but the content of a main functional delivery agent, i.e., caprylocaproyl macrogolglyceride, is up to 5% and a special drug administration instrument is generally required to achieve spray administration of the drug.

The brain targeting property is one of the potential advantages of nasal administration, e.g., US2021087250A points out that nasal administration of polypeptide drugs is often one of the main purposes for achieving brain targeting. In addition, US2011129462A1 has experimentally demonstrated that nasal absorption enhancers such as DDM can achieve targeted delivery of polypeptide compounds such as antibody fragments to the brain. The instructions of the FDA-approved hypoglycemic version of semaglutide product (trade name: Ozempic) point out that the hypoglycemic effect of semaglutide mainly depends on its regulation to insulin secretion, and thus can be exerted without entering the central nervous system (CNS). Although the instructions of the FDA-approved weight-loss version of semaglutide product (trade name: Wegovy) point out that semaglutide may exert its weight-loss effect by activating a GLP-1 receptor in the CNS that regulates appetite, but other action mechanisms (e.g., delayed gastric emptying) are not excluded. Considering the adverse effects that may affect risk-benefit characteristics after the drug enters the CNS, for example, Jia-Rui Li, et al. reported two cases of adverse reactions to depression due to the use of semaglutide in the article *"Semaglutide-associated depression: a report of two cases",* the authors attribute such adverse effects to semaglutide that activates a GLP-1 receptor located in the CNS. Therefore, the brain-targeting properties of drugs such as GLP-1 receptor agonists can be avoided or reduced to facilitate improving their risk-benefit characteristics.

This field requires technical solutions to enable nasal administration of GLP-1 receptor agonists, including semaglutide, and to reduce/avoid their brain-targeted delivery.

### SUMMARY OF THE INVENTION

One of the objects of the present invention is to provide composition for nasal administration comprises a GLP-1 receptor agonist. The composition can also avoid delivery targeting a central nervous system.

In order to fulfill the above objects, the present invention provides a composition comprises a GLP-1 receptor agonist and a nasal absorption enhancer, wherein the GLP-1 receptor agonist is selected from one of liraglutide, semaglutide and tirzepatide; and the nasal absorption enhancer is selected from one or more of dodecyl-β-D-maltoside, nonyl-β-D-glucopyranoside, polyoxyethylene (7) dodecyl ether, polyoxyethylene (8) dodecyl ether, polyoxyethylene (9) dodecyl ether, polyethylene glycol octylphenyl ether, sodium dodecyl sulfate, polysorbate-80, diethylene glycol monoethyl ether, polyethylene glycol 400, polysorbate-20, ethoxylated C16-18-ol, polyoxyethylene (10) cetyl ether, polyoxyethylene (10) octadecyl ether, polyoxyethylene (10) oleyl ether, polyoxyethylene (20) oleyl ether, n-octyl-β-D-glucopyranoside, 1-O-decyl-β-D-maltoside and polyoxyethylene (2) cetyl ether. Another object of the present invention is to provide a method for preparing the composition of the present invention, wherein the method comprises: mixing a GLP-1 receptor agonist with a nasal absorption enhancer.

Another object of the present invention is to provide use of the composition of the present invention in the preparation of a drug for the treatment of a disease, wherein the disease is selected from one or more of type 2 diabetes mellitus, overweight or obesity.

Both in vivo and in vitro tests show that the composition of the present invention has good stability, good bioavailability after nasal administration in rats, and minimal drug distribution in brain tissues.

### DESCRIPTION OF THE DRAWINGS

FIG. 1 is a HPLC chromatogram of Formulation C1 measured at 50°C on Day 10 in Example 3.
FIG. 2 is a HPLC chromatogram of Formulation C2 measured at 50°C on Day 10 in Example 3.
FIG. 3 is a HPLC chromatogram of Formulation C3 measured at 50°C on Day 10 in Example 3; and
FIG. 4 is a HPLC chromatogram of Formulation C4 measured at 50°C on Day 10 in Example 3.

### DETAILED DESCRIPTION OF THE INVENTION

In a first aspect, the present invention provides a composition comprises a GLP-1 receptor agonist and a nasal absorption enhancer, wherein the GLP-1 receptor agonist is selected from one of liraglutide, semaglutide and tirzepatide; and the nasal absorption enhancer is selected from one or more of dodecyl-β-D-maltoside, nonyl-β-D-glucopyranoside, polyoxyethylene (7) dodecyl ether, polyoxyethylene (8) dodecyl ether, polyoxyethylene (9) dodecyl ether, polyethylene glycol octylphenyl ether, sodium dodecyl sulfate, polysorbate-80, diethylene glycol monoethyl ether, polyethylene glycol 400, polysorbate-20, ethoxylated C16-18-ol, polyoxyethylene (10) cetyl ether, polyoxyethylene (10) octadecyl ether, polyoxyethylene (10) oleyl ether, polyoxyethylene (20) oleyl ether, n-octyl-β-D-glucopyranoside, 1-O-decyl-β-D-maltoside and polyoxyethylene (2) cetyl ether. According to some embodiments of the present invention, the composition is a liquid.

According to some embodiments of the present invention, the composition uses water as a solvent. According to some embodiments of the present invention, the GLP-1 receptor agonist is semaglutide. According to some embodiments of the present invention, the nasal absorption enhancer is one or more of dodecyl-β-D-maltoside and enhancerthose having a molecular formula of CH₃(CH₂)ₙ₋₁[OCH₂CH₂]ₘOH, wherein n and m are integers. According to some embodiments, n is an integer selected from 10-16; and m is an integer selected from 4-10.

According to some embodiments of the present invention, the nasal absorption enhancer is selected from dodecyl-β-D-maltoside, polyoxyethylene (7) dodecyl ether, polyoxyethylene (8) dodecyl ether and polyoxyethylene (9) dodecyl ether. According to some embodiments, the nasal absorption enhancer is selected from dodecyl-β-D-maltoside or polyoxyethylene (9) dodecyl ether. According to some embodiments of the present invention, the content of the GLP-1 receptor agonist in the composition is 0.28 mg/mL to 170.0 mg/mL. In some embodiments, the content of the GLP-1 receptor agonist in the composition is 17.0 mg/mL to 70.0 mg/mL. In some embodiments, the content of the GLP-1 receptor agonist in the composition is 5.25 mg/mL to 22.2 mg/mL. In some embodiments, the content of the GLP-1 receptor agonist in the composition is 5.25 mg/mL to 10.5 mg/mL.

According to some embodiments of the present invention, the w/v% content of the nasal absorption enhancer in the composition is 0.25% to 2.5%. In some embodiments, the w/v% content of the nasal absorption enhancer in the composition is 0.25% to 1.0%. In some embodiments, the w/v% content of the nasal absorption enhancer in the composition is 0.25% to 0.5%.

According to some embodiments of the present invention, a mass ratio of the nasal absorption enhancer to the GLP-1 receptor agonist in the composition is 0.01 to 18. In some embodiments, a mass ratio of the nasal absorption enhancer to the GLP-1 receptor agonist in the composition is 0.03 to 1.50.

According to some embodiments of the present invention, the composition further comprises a preservative. In some embodiments, the preservative is selected from phenol, benzalkonium chloride, benzylammonium chloride, 2-trichloromethyl-2-propanol, butyl p-hydroxybenzoate, propyl p-hydroxybenzoate, benzethonium chloride, chlorocresol, and benzoic acid.

In another aspect, the present invention provides a method for preparing the composition of the present invention, wherein the method comprises: mixing a GLP-1 receptor agonist with a nasal absorption enhancer.

In another aspect, the present invention provides use of the composition of the present invention in the preparation of a drug for the treatment of a disease, wherein the disease is selected from one or more of type 2 diabetes mellitus, overweight or obesity.

### Examples

### Example 1: parallel artificial membrane permeability assay (PAMPA) test of test formulations

**1.1. Test objective:** to investigate the differences in in-vitro permeability of semaglutide having a concentration of 10 mM in pH7.4 PrismaHT buffer by using the PAMPA method after the addition of different types and proportions of permeation promoters.

### 1.2. Test method

### (1) Compositions of Formulations 1-20

An appropriate amount of 10 mM semaglutide mother liquor was weighed and placed in a liquid phase injection vial, and different permeation promoters were weighed and added correspondingly according to types and proportions listed in Table 1; a magnetic stirrer was added; the magnetic stirrer was turned on to adjust a rotation speed to 650 rpm; and the temperature of the magnetic stirrer was kept at 37°C, followed by stirring for later use.

**Table 1**

| Formulation Nos. | Nasal absorption enhancer and dosage | | |
|---|---|---|---|
| | Name | Abbreviated as | Dosage |
| 1 | Dodecyl-β-D-maltoside | DDM | 0.25% |
| 2 | Nonyl-β-D-glucopyranoside | NG | 0.25% |
| 3 | Polyoxyethylene (8) dodecyl ether | C12E8 | 0.25% |
| 4 | Polyoxyethylene (8) dodecyl ether | C12E8 | 0.5% |
| 5 | Polyoxyethylene (8) dodecyl ether | C12E8 | 1.00% |
| 6 | Polyethylene glycol octylphenyl ether | TritonX-100 | 0.25% |
| 7 | Sodium dodecyl sulfate | SDS | 0.25% |
| 8 | Polysorbate-80 | Tween 80 | 0.25% |
| 9 | Diethylene glycol monoethyl ether | Transcutol HP | 0.25% |
| 10 | Polyethylene glycol 400 | PEG 400 | 0.25% |
| 11 | Polysorbate-20 | Tween 20 | 0.25% |
| 12 | Ethoxylated C16-18-ol | NA | 0.25% |
| 13 | Polyoxyethylene (10) cetyl ether | Brij56 | 0.25% |
| 14 | Polyoxyethylene (10) octadecyl ether | C18E10 | 0.25% |
| 15 | Polyoxyethylene (10) oleyl ether | Brij O10 | 0.25% |
| 16 | Polyoxyethylene (20) oleyl ether | Brij O20 | 0.25% |
| 17 | n-Octyl-β-D-glucopyranoside | OGP | 0.25 |
| 18 | 1-O-decyl-β-D-maltoside | DM | 0.25% |
| 19 | Polyoxyethylene (9) dodecyl ether | C12E9 | 0.25% |
| 20 | Polyoxyethylene (2) cetyl ether | Brij 52 | 0.25% |

### (2) PAMPA test

Donor wells solutions were solutions of Formulations 1-20;
An acceptor wells solution was pH 7.4 Prisma HT buffer.
   1) PAMPA membrane hydration: a Hydration Solution (Pion) was prepared (used at room temperature); donor wells of a PAMPA plate were carefully removed; 200 µl of Hydration Solution was added to each well, and whether each empty surface forms a curved surface due to surface tension was observed; and acceptor wells of the PAMPA plate were covered carefully, while air bubbles were avoided on contact surfaces. The Hydration Solution was kept moist and hydrated overnight, and the hydrated membrane will appear translucent or have white spots. At this point, a PAMPA experiment can begin.
   2) Operations were performed on the PAMPA plate, and each formulation was divided into 4 samples in parallel, which were added with 200 µl of corresponding donor wells solution at the bottom, respectively.
   3) The membrane was gently covered on the acceptor wells solution to avoid the formation of air bubbles in the middle (if more air bubbles were generated, it can be placed in a vacuum drying oven at 25°C and vacuumized for 2-3 times).
   4) 200 µl of each corresponding acceptor wells solution was added to acceptor wells, taking care to add the solution to the membrane to avoid the formation of cavities.
   5) After incubation at 37°C for 5 h, the donor wells solution and the acceptor wells solution were taken and the samples were processed.

### (3) Sample processing

100 µl of each sample in the donor wells, in the acceptor wells and at 0 h was taken, diluted with 900 µl of 50% acetonitrile, and then centrifuged according to centrifugation parameters: 13000 rmp, 25°C, 5 min. 200 µl of supernatant was taken and added into a liquid phase injection vial and delivered for analysis for HPLC detection, and conditional parameters of HPLC were shown in Table 2.

**Table 2**

| Compound name | Semaglutide | | | |
|---|---|---|---|---|
| Mobile phase A | 100 mM ammonium dihydrogen phosphate solution | | | |
| Mobile phase B | Acetonitrile solution | | | |
| Chromatographic column | ACE Bioanalytical 300A C18 150*4.6mm*3um | | | |
| Wavelength | 220nm&284nm | | | |
| Liquid phase | Agilent 1260 InfinityII. | | | |
| Injection volume | 2 µL | | | |
| Sample disk temperature | 8°C | | | |
| Column temperature | 30°C | | | |
| Isocratic | Time(min) | Flow rate(mL/min) | A(%) | B(%) |
| | 0-5 | 1.0 | 55 | 45 |

### (4) Calculation formula

$R = 1 - \frac{{C}_{D} \left(t\right)}{{C}_{D} \left(0\right)} - \frac{{V}_{A}}{{V}_{D}} \frac{{C}_{A} \left(t\right)}{{C}_{D} \left(0\right)}$ $log Pe = log \left[-\frac{2.303 {V}_{D}}{A \left(t-{t}_{LAG}\right)}\left(\frac{{V}_{A}}{{V}_{A} + {V}_{D}}\right)log\left[1-\left(\frac{{V}_{A} + {V}_{D}}{{V}_{D} \left(1-R\right)}\right)\frac{{C}_{A} \left(t\right)}{{C}_{D} \left(0\right)}\right]\right]$
VA-the volume in the acceptor wells (ml);
VD-the volume in the donor wells (ml);
A-the filtration area (cm2);
t-the incubation time (s);
tLAG-the steady-state time (s);
CD(t)-the concentration of the compound in donor well at time t (lM);
CA(t)-the concentration of the compound in acceptor well at time t (lM);
CD(0)-the concentration of the compound in donor well at time 0 (lM).

### 1.3. Statistics of PAMPA test results

The test results were shown in Table 3.

**Table 3**

| Formulation Nos. | Formulations | Concentration at 0 h (mg/ml) | Average concentration of donor wells solution at 5 h (mg/ml) | Average concentration of acceptor wells solution at 5h (mg/ml) | Donor wells percentage | Transmission percentage | LogPe | Pe(10⁻⁶cm/s) |
|---|---|---|---|---|---|---|---|---|
| P1 | +0.25% dodecyl-β-D-maltoside (DDM) | 4.1613 | 3.860 | 0.034 | 92.8 | 0.8 | - 6.45 | 0.35 |
| P2 | +0.25% nonyl-β-D-glucopyranoside (NG) | 4.1253 | 3.806 | 0.008 | 92.3 | 0.2 | - 7.06 | 0.09 |
| P3 | +0.25% polyoxyethylene (8) dodecyl ether (C12E8) | 3.9326 | 3.585 | 0.041 | 91.2 | 1.0 | - 6.35 | 0.45 |
| P4 | +0.50% polyoxyethylene (8) dodecyl ether (C12E8) | 4.0167 | 3.333 | 0.058 | 83.0 | 1.5 | - 6.16 | 0.69 |
| P5 | +1.00% polyoxyethylene (8) dodecyl ether (C12E8) | 4.0557 | 3.480 | 0.037 | 85.8 | 0.9 | - 6.37 | 0.42 |
| P6 | +0.25% polyethylene glycol octylphenyl ether (Triton X-100) | 3.9121 | 3.556 | 0.017 | 90.9 | 0.4 | - 6.73 | 0.19 |
| P7 | +0.25% sodium dodecyl sulfate (SDS) | 4.0913 | 3.669 | 0.009 | 89.7 | 0.2 | - 7.03 | 0.09 |
| P8 | +0.25% polysorbate-80 (Tween 80) | 3.8413 | 3.635 | 0.008 | 94.6 | 0.2 | - 7.07 | 0.08 |
| P9 | +0.25% diethylene glycol monoethyl ether (Transcutol HP) | 4.0353 | 3.766 | 0.001 | 93.3 | 0.0 | - 7.87 | 0.01 |
| P10 | +0.25% polyethylene glycol 400 (PEG 400) | 3.9531 | 3.686 | 0.003 | 93.2 | 0.1 | - 7.48 | 0.03 |
| P11 | +0.25% polysorbate-20 (Tween 20) | 3.8963 | 3.603 | 0.035 | 92.5 | 0.9 | - 6.42 | 0.38 |
| P12 | +0.25% Ethoxylated C16-18-ol | 3.7370 | 3.472 | 0.002 | 92.9 | 0.1 | - 7.60 | 0.03 |
| P13 | +0.25% polyoxyethylene (10) cetyl ether (Brij56) | 4.0480 | 3.736 | 0.028 | 92.3 | 0.7 | - 6.53 | 0.29 |
| P14 | +0.25% polyoxyethylene (10) octadecyl ether (C18E10) | 4.0810 | 3.701 | 0.017 | 90.7 | 0.4 | - 6.73 | 0.18 |
| P15 | +0.25% polyoxyethylene (10) oleyl ether (Brij O10) | 3.9486 | 3.582 | 0.019 | 90.7 | 0.5 | - 6.65 | 0.22 |
| P16 | +0.25% polyoxyethylene (20) oleyl ether (Brij O20) | 4.0910 | 4.062 | 0.004 | 99.3 | 0.1 | - 7.36 | 0.04 |
| P17 | +0.25% n-octyl-β-D-glucopyranoside (OGP) | 3.9294 | 3.670 | 0.003 | 93.4 | 0.1 | - 7.44 | 0.04 |
| P18 | +0.25% 1-O-decyl-β-D-maltoside (DM) | 4.0348 | 4.051 | 0.001 | 100.4 | 0.0 | - 7.84 | 0.01 |
| P19 | +0.25% polyoxyethylene (9) dodecyl ether (C12E9) | 3.8997 | 3.535 | 0.024 | 90.7 | 0.6 | - 6.56 | 0.27 |
| P20 | +0.25% polyoxyethylene (2) cetyl ether (Brij 52) | 4.0969 | 3.818 | 0.006 | 93.2 | 0.1 | - 7.21 | 0.06 |
| P21 | +0.5% dodecyl-β-D-maltoside (DDM) | 4.0514 | 3.788 | 0.036 | 93.5 | 0.9 | - 5.75 | 0.42 |
| P22 | +1% dodecyl-β-D-maltoside (DDM) | 3.9985 | 3.826 | 0.034 | 95.7 | 0.6 | - 7.52 | 0.15 |
| P23 | +0.5% polyoxyethylene (9) dodecyl ether (C12E9) | 4.0004 | 3.572 | 0.032 | 89.3 | 0.8 | - 5.45 | 0.31 |
| P24 | +1% polyoxyethylene (9) dodecyl ether (C12E9) | 3.9952 | 3.767 | 0.029 | 94.3 | 0.5 | - 7.85 | 0.11 |

In the parameters in the above table, the smaller the donor wells percentage, the larger the transmission percentage, the larger the LogPe value (closer to a positive value), and the larger the Pe value, indicating that the greater the permeation amount, and the better the permeability of the formulation in relative terms.

### 1.4. Conclusion

According to the in-vitro permeation data of Skin PAMPA, under the dosages of permeation promoters having the same concentration of 0.25% (w/v%), dodecyl-β-D-maltoside (DDM), polyoxyethylene (8) dodecyl ether (C12E8) and polysorbate-20 (Tween 20) had better permeation promoting effects, and the dosage of the permeation promoter was appropriately increased, e.g., to 0.50% (w/v%), such that the permeation promoting effect was improved.

The permeation promoter with good permeation promoting effect was selected from a PAMPA experiment, configured as a nasal drip preparation. After nasal administration in SPF-grade rats, the drug concentrations in plasma and brain tissues of the rats were measured over time.

### Example 2. Pharmacokinetic test - determination of bioavailability

### 2.1. Test materials and test objects

**(1) Animal:** SD rats (SPF grade), 7-8 weeks old, among which a female rat has a weight of 210-230 g and a male fat has a weight of 260-290 g. 4 rats in each group, half male and half female.
**(2) Test formulation:** as shown in Table 4

**Table 4**

| Serial number^{a} | Nasal absorption enhancer and its dosage^{b} | Semaglutide concentration | Proposed administration route | Proposed administration dosage |
|---|---|---|---|---|
| F4 | 0.25% dodecyl-β-D-maltoside (DDM) | 5.25 mg/ml | Nasal drip | 0.1 mL/kg |
| F5 | 0.5% dodecyl-β-D-maltoside (DDM) | 5.25 mg/ml | Nasal drip | 0.1 mL/kg |
| F6 | 0.5% polyoxyethylene (7) dodecyl ether (C12E7) | 5.25 mg/ml | Nasal drip | 0.1 mL/kg |
| F7 | 0.5% polyoxyethylene (8) dodecyl ether (C12E8) | 5.25 mg/ml | Nasal drip | 0.1 mL/kg |
| F8 | 0.5% polyoxyethylene (9) dodecyl ether (C12E9) | 5.25 mg/ml | Nasal drip | 0.1 mL/kg |
| F9 | 2.5% polysorbate-20 (Tween20) | 5.25 mg/ml | Nasal drip | 0.1 mL/kg |
| F10 | 0.5% polyoxyethylene (10) cetyl ether | 5.25 mg/ml | Nasal drip | 0.1 mL/kg |
| F11 | 0.25% dodecyl-β-D-maltoside (DDM) | 10.5 mg/mL | Nasal drip | 0.1 mL/kg |
| F12 | 0.25% polyoxyethylene (9) dodecyl ether (C12E9) | 10.5 mg/mL | Nasal drip | 0.1 mL/kg |
| F13 | 0.25% dodecyl-β-D-maltoside (DDM) | 22.2 mg/mL | Nasal drip | 0.1 mL/kg |
| F14 | 0.25% polyoxyethylene (9) dodecyl ether (C12E9) | 22.2 mg/mL | Nasal drip | 0.1 mL/kg |
| F15 | 0.50% dodecyl-β-D-maltoside (DDM) | 10.5 mg/mL | Nasal drip | 0.1 mL/kg |
| F16 | 0.50% polyoxyethylene (9) dodecyl ether (C12E9) | 10.5 mg/mL | Nasal drip | 0.1 mL/kg |
| F18 | 0.25% polyoxyethylene (9) dodecyl ether (C12E9) | 5.25 mg/mL | Nasal drip | 0.1 mL/kg |
| F19 | 0.5% dodecyl-β-D-maltoside (DDM) (pH6.0) | 5.25 mg/mL | Nasal drip | 0.1 mL/kg |
| F20 | 0.5% polyoxyethylene (9) dodecyl ether (C12E9) (pH6.0) | 5.25 mg/mL | Nasal drip | 0.1 mL/kg |
| F21 | N/A | 0.0225 mg/mL | Subcutaneous injection | 1 mL/kg |
| F22 | N/A | 0.045 mg/mL | Subcutaneous injection | 1 mL/kg |
| F23 | N/A | 0.09 mg/mL | Subcutaneous injection | 1 mL/kg |

| | | | | |
|---|---|---|---|---|
| Note: ^{a} Each formulation contained 0.55 g/100 ml phenol as a bacteriostatic agent. ^{b} The concentration of each nasal absorption enhancer was w/v% relative to the whole formulation. | | | | |

### 2.2. Method

**Administration:** all formulations underwent single administration, with the administration routes and dosages shown in Table 4.

**Sampling:** blood samples were collected before administration (0 h) and at 0.083 h, 0.5 h, 0.75 h, 1 h, 2 h, 4 h, 6 h, 8 h, 12 h, and 24 h after administration. Blood was collected from the post-glomus venous plexus. About 0.20 mL of each sample was collected and anticoagulated with EDTA. Each blood sample was placed on ice after collection. The plasma was separated by centrifugation within 2 h (centrifugation conditions: 3500 rpm, 10 min, 2-8°C).

**Determination conditions:** conditional parameters for HPLC-MS were shown in Table 5.

**Table 5**

| Compound name | Semaglutide | | | |
|---|---|---|---|---|
| Liquid phase method | | | | |
| Mobile phase A | 0.5% formic acid water | | | |
| Mobile phase B | Methanol:acetonitrile = 3:7 (0.5% formic acid) | | | |
| Probe wash | Acetonitrile:methanol:water = 3:3:4 (0.2% formic acid) | | | |
| Chromatographic column | ACE3C850*2.1mm3µm | | | |
| Internal standard | Tolbutamide | | | |
| Liquid phase | Shimadzu exionLC30AD | | | |
| Autosampler | Exion LC AD Multiplate Sampler | | | |
| Injection volume | 5 µL | | | |
| Gradient | Time(min) | Flow rate(mL/min) | A(%) | B(%) |
| | 0 | 0.4 | 45 | 55 |
| | 2.5 | 0.4 | 5.0 | 95 |
| | 4.0 | 0.4 | 5.0 | 95 |
| | 4.1 | 0.4 | 45 | 55 |
| | 5.5 | 0.4 | 45 | 55 |

| Mass spectrometry | | | | |
|---|---|---|---|---|
| Mass spectrum | TRIPLEQUAD^{™}6500⁺, AB | | | |
| Ionization source | Electro-spray ionization (ESI) source | | | |
| Scan mode | MRM | | | |
| Polarity | Positive | | | |
| Compound name | Ion pair (m/z) | Retention time (min) | Declustering voltage (eV) | Collision energy (eV) |
| Semaglutide | 1029.3/1238.2 | 1.22 | 80 | 45 |
| (Internal standard) | 271.1/155.0 | 0.82 | 50 | 24 |
| Mass spectrum parameters | Curtain gas (psi): | | 20 | |
| | Atomized gas (psi): | | 35 | |
| | Auxiliary heating gas (psi): | | 40 | |
| | Ion transmission voltage (V): | | 5500 | |
| | 3Atomization temperature (°C): | | 450 | |
| | Collision gas: | | 8 | |
| | Entry voltage (eV): | | 10 | |
| | Collision chamber exit voltage (eV): | | 11 | |

### 2.3. Results

Main pharmacokinetic parameters of each formulation were shown in Table 6.

**Table 6**

| Formulation Nos. | Tₘₐₓ(h) | Cₘₐₓ(ng/mL) | AUC₍₀₋ₜ₎(ng·h/mL) | Relative bioavailability (%) |
|---|---|---|---|---|
| F4 | 1.0±0.0 | 89±153 | 869±1564 | 2.68 |
| F5 | 1.3±0.5 | 170±230 | 1817±2727 | 5.60 |
| F6 | 1.8±0.5 | 124±73.3 | 1296±838 | 3.99 |
| F7 | 1.5±0.6 | 96.4±45.3 | 915±532 | 2.82 |
| F8 | 1.0±0.0 | 194±48 | 1996±887 | 6.15 |
| F9 | 2.0±0.0 | 45.5±89 | 469±936 | 1.44 |
| F10 | 1.3±0.5 | 117±62.9 | 1076±571 | 3.31 |
| F11 | 0.81±0.239 | 23.76±21.1 | 141±137 | 0.22 |
| F12 | 0.54±0.529 | 14.98±5.18 | 72.60±43.0 | 0.11 |
| F13 | 0.40±0.21 | 13.06±14.9 | 73.49±113 | 0.05 |
| F14 | 1.00±0.00 | 11.52±3.63 | 59.53±25.18 | 0.04 |
| F15 | 1.02±0.783 | 105±140 | 722±1022 | 1.11 |
| F16 | 0.88±0.144 | 118±108 | 915±973 | 1.41 |
| F18 | 0.94±0.13 | 23.51±14.78 | 112±78.90 | 0.34 |
| F19 | 1.00±0.00 | 105.48±92 | 735±676 | 2.26 |
| F20 | 0.94±0.13 | 73.90±33.3 | 527±319 | 1.62 |
| F21 | 7.00±1.15 | 110±24.6 | 1926±375 | N/A |
| F22 | 4.00±1.63 | 157±30.8 | 2783±676 | N/A |
| F23 | 6.75±4.57 | 323±92.2 | 5804±1289 | N/A |

| | | | | |
|---|---|---|---|---|
| ***Note: "relative bioavailability (F)" was defined as: F = (AUCT*DR)/(AUCR*DT)*100%** **AUCT: area under the plasma concentration-time curve of nasal drip preparation;** **DR: administration dosage of hypodermic needle;** **AUCR: area under the plasma concentration-time curve of hypodermic needle; and** **DT: administration dosage of nasal drip formulation.** | | | | |

**The results of the above animal experiments showed that different concentrations of dodecyl-β-D-maltoside (DDM) and polyoxyethylene (9) dodecyl ether (C12E9) can be well absorbed into the blood when combined with different concentrations of semaglutide.**

### Example 3. Compatibility/stability studies

The changes in semaglutide content and impurities in a semaglutide solution, semaglutide+different permeation promoter solutions, and semaglutide+different bacteriostatic agent solutions were determined under HPLC conditions in Example 1 after being stored in the dark for 10 days (10 D) at 40°C and 50°C, and the results were shown in Table 7.

**Table 7**

| Formulations | | C1 | C2 | C3 | C4 | C5 | C6 | C7 | |
|---|---|---|---|---|---|---|---|---|---|
| Formulation design | | SEM solution | SEM solution +1% DDM | SEM solution +1% C12E9 | SEM solution + 0.1% benzalkonium chloride | SEM solution + 0.1% benzoic acid | SEM solution +0.18% sodium methylparaben +0.02% sodium methylparaben | SEM solution +0.02% phenol | |
| Main peak purity, % | 0 h | 99.9 6 | 99.81 | 99.92 | 99.92 | 99.92 | 99.93 | 99.95 | |
| | 40°C, 5D | 98.42 | 99.47 | 99.53 | 98.68 | 97.91 | 98.55 | 99.45 | |
| | | 50°C, 5D | 97.9 | 98.62 | 97.04 | 98.23 | 96.84 | 98.21 | 99.16 |
| | | 40°C, 10D | 94.3 1 | 98.2 | 98.36 | 97.1 | 94.03 | 93.48 | 98.29 |
| | | 50°C, 10D | 93.1 | 94.45 | 96.15 | 93.54 | 90.9 | 90.17 | 95.92 |
| Impurity, % | Hydrophilic impurity 1, % | 0 H | / | / | / | / | / | / | / |
| | | 40°C, 5D | / | / | / | / | / | / | / |
| | | 50°C, 5D | / | / | 0.47 | / | / | / | / |
| | | 40°C, 10D | 0.06 | / | / | 0.05 | 0.05 | 2.28 | / |
| | | 50°C, 10D | 0.09 | 0.04 | 0.01 | 0.12 | 0.12 | 4.23 | 0.05 |
| | Hydrophilic impurity 2, % | 0 H | 0.04 | 0.04 | 0.08 | 0.08 | 0.08 | 0.07 | 0.05 |
| | | 40°C, 5D | 0.8 | 0.28 | 0.38 | 1.23 | 1.23 | 0.84 | 0.36 |
| | | 50°C, 5D | 1.35 | 0.7 | 1.04 | 1.75 | 1.75 | 1.09 | 0.67 |
| | | 40°C, 10D | 3.36 | 0.87 | 1.23 | 3.51 | 3.51 | 2.4 | 0.91 |
| | | 50°C, 10D | 4.08 | 2.79 | 2.56 | 5.26 | 5.26 | 3.21 | 2.1 |
| | Hydrophobic impurity, % | 0 H | / | 0.15 | / | / | / | / | / |
| | | 40°C, 5D | 0.78 | 0.24 | 0.09 | 0.86 | 0.86 | 0.63 | 0.19 |
| | | 50°C, 5D | 0.76 | 0.68 | 1.45 | 1.41 | 1.41 | 0.69 | 0.18 |
| | | 40°C, 10D | 2.29 | 0.94 | 0.42 | 2.4 | 2.4 | 1.85 | 0.8 |
| | | 50°C, 10D | 2.74 | 2.72 | 1.28 | 3.74 | 3.74 | 2.37 | 1.94 |

| | | | | | | | | | |
|---|---|---|---|---|---|---|---|---|---|
| Note: SEM was semaglutide, and each formulation was an aqueous solution, wherein the concentration of SEM was 5.252 mg/ml. | | | | | | | | | |

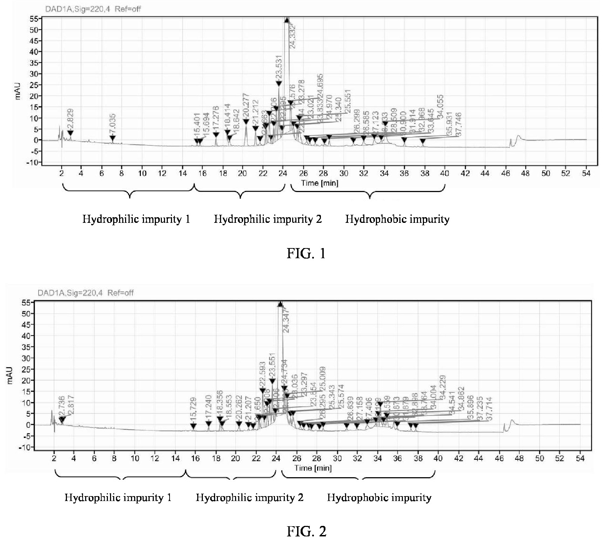

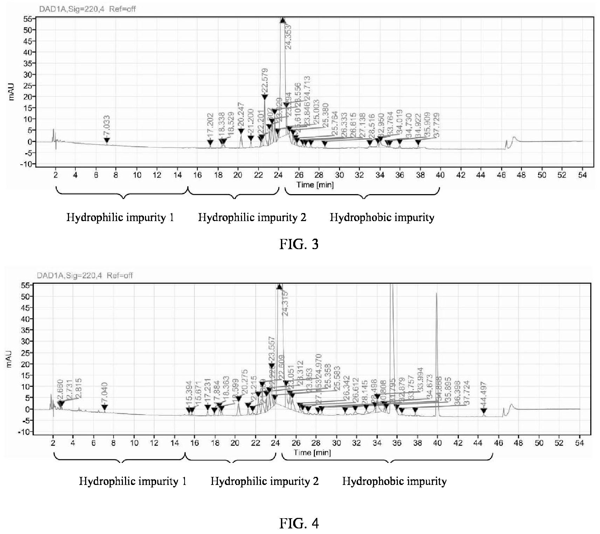

The definitions of the hydrophilic impurities 1 and 2 and the hydrophobic impurity in Table 7 were shown in HPLC chromatograms of Formulations C1 to C4 shown in FIGS. 1-4 determined at 50°C on Day 10.

As a control, main peak intensities of compositions J and K in CN116942612A at 50°C on Day 5 were determined using the same method, both of which were below 95%.

Results: both permeation promoters had protective effects on semaglutide and inhibited the degradation of semaglutide; and benzalkonium chloride, as a bacteriostatic agent in nasal spray preparations, also had a protective effect on semaglutide in this product.

### Example 4. Pharmacokinetic test - brain tissue distribution

### 4.1. Test materials and test objects

**(1) Animal:** the same as Example 2. Each test formulation group included 40 rats.
**(2) Test formulations:** F5, F8 and F23 in Example 2.

### 4.2. Method

**Administration:** the same as Example 2.

**Sampling:** 5 male and 5 female rats were dislocated at 1, 8, 16 and 24 h after administration, respectively; the brains were taken, and quickly rinsed with clear water; after precision weighing, the tissues were minced and mixed well with pre-cooled normal saline at 1 g:3 mL, then homogenized by an ultrasonic cell grinder, and centrifuged at 12000 rpm for 2 min; and a supernatant was stored at -20°C for testing. A detection concentration (ng/mL) of semaglutide in tissue homogenate was divided by the density (g tissue/mL) of the tissue homogenate to calculate a tissue concentration in ng/g tissues.

**Detection:** the conditions were the same as those in Example 2.

### 4.3. Results

Results were shown in Table 8.

**Table 8**

| Determination time (h) | Content in brain tissues (ng/g) | | |
|---|---|---|---|
| | F5 | F8 | F23 |
| 1 | BLQ | BLQ | BLQ |
| 8 | BLQ | BLQ | BLQ |
| 16 | BLQ | BLQ | 0.33 |
| 24 | BLQ | BLQ | 0.69 |

BLQ indicated being below a detection limit

As shown in Table 8, the distribution amounts of F5 and F8 in the present invention in rat brain parts after nasal administration were significantly lower than that of F23 after injection administration.

### Example 5. Pharmacokinetic - brain tissue distribution

The method was the same as Example 4, and the types of test formulations were increased, that is, Formulations F6, F7, F9, F10, F13, and F14 selected from Table 6 were tested.

Semaglutide in Formulations F6, F7, F9, and F10 was found in brain tissues under at least one time point, among which the dosages of semaglutide in F6, F7 and F10 were the same as those of F5 and F8, but the types of nasal absorption enhancers were different, indicating that there was great uncertainty about whether the nasal absorption enhancers can avoid brain delivery.

In addition, F13 and F14 in highest dosages were not detected at any time point for brain drug analysis.

## Claims

1. A composition comprises a GLP-1 receptor agonist and a nasal absorption enhancer, wherein the GLP-1 receptor agonist is selected from one of liraglutide, semaglutide and tirzepatide; and the nasal absorption enhancer is selected from one or more of dodecyl-β-D-maltoside, nonyl-β-D-glucopyranoside, polyoxyethylene (7) dodecyl ether, polyoxyethylene (8) dodecyl ether, polyoxyethylene (9) dodecyl ether, polyethylene glycol octylphenyl ether, sodium dodecyl sulfate, polysorbate-80, diethylene glycol monoethyl ether, polyethylene glycol 400, polysorbate-20, ethoxylated C16-18-ol, polyoxyethylene (10) cetyl ether, polyoxyethylene (10) octadecyl ether, polyoxyethylene (10) oleyl ether, polyoxyethylene (20) oleyl ether, n-octyl-β-D-glucopyranoside, 1-O-decyl-β-D-maltoside and polyoxyethylene (2) cetyl ether.

2. The composition according to claim 1, wherein the composition is a liquid.

3. The composition according to claim 2, wherein the composition uses water as a solvent.

4. The composition according to claim 1, wherein the GLP-1 receptor agonist is semaglutide.

5. The composition according to claim 1, wherein the nasal absorption enhancer is selected from one or more of dodecyl-β-D-maltoside and an absorption enhancer having a molecular formula of CH₃(CH₂)ₙ₋₁[OCH₂CH₂]ₘOH, wherein n and m are integers.

6. The composition according to claim 5, wherein n is an integer selected from 10-16; and m is an integer selected from 4-10.

7. The composition according to claim 1, wherein the nasal absorption enhancer is selected from dodecyl-β-D-maltoside, polyoxyethylene (7) dodecyl ether, polyoxyethylene (8) dodecyl ether and polyoxyethylene (9) dodecyl ether, preferably dodecyl-β-D-maltoside or polyoxyethylene (9) dodecyl ether.

8. The composition according to claim 1, wherein the content of the GLP-1 receptor agonist in the composition is 0.28 mg/mL to 170.0 mg/mL.

9. The composition according to claim 8, wherein the content of the GLP-1 receptor agonist in the composition is 17.0 mg/mL to 70.0 mg/mL.

10. The composition according to claim 8, wherein the content of the GLP-1 receptor agonist in the composition is 5.25 mg/mL to 22.2 mg/mL, preferably 5.25 mg/mL to 10.5 mg/mL.

11. The composition according to claim 1, wherein the w/v% content of the nasal absorption enhancer in the composition is 0.25% to 2.5%.

12. The composition according to claim 11, wherein the w/v% content of the nasal absorption enhancer in the composition is 0.25% to 1.0%, preferably 0.25% to 0.5%.

13. The composition according to claim 1, wherein a mass ratio of the nasal absorption enhancer to the GLP-1 receptor agonist in the composition is 0.03 to 1.50.

14. The composition according to claim 1, wherein the composition further comprises a preservative, wherein the preservative is, for example, phenol, benzalkonium chloride, benzylammonium chloride, 2-trichloromethyl-2-propanol, butyl p-hydroxybenzoate, propyl p-hydroxybenzoate, benzethonium chloride, chlorocresol, and benzoic acid, preferably benzalkonium chloride.

15. Use of the composition according to any one of claims 1 to 14 in the preparation of a drug for the treatment of a disease, wherein the disease is selected from one or more of type 2 diabetes mellitus, overweight or obesity.
